Europäisches Patentamt

(19) European Patent Office

Office européen des brevets

(11) Publication number: 0 392 546
A2

## EUROPEAN PATENT APPLICATION

(21) Application number: 90107066.4

(22) Date of filing: 12.04.90

(51) Int. Cl.⁵: C12Q 1/68

(30) Priority: 14.04.89 YU 767/89

(43) Date of publication of application:
17.10.90 Bulletin 90/42

(84) Designated Contracting States:
AT BE CH DE DK ES FR GB GR IT LI LU NL SE

(71) Applicant: RO INSTITUT ZA MOLEKULARNU
GENETIKU I GENETICKO INZENJERSTVO
Vojvode Stepe 283/III
YU-11001 Beograd(YU)

(72) Inventor: Drmanac, Radoje T.
Zvecanska 46
YU-11000 Belgrade(YU)
Inventor: Crkvenjakov, Radomir B.
Bulevar JNA 118
YU-11000 Belgrade(YU)

(74) Representative: von Füner, Alexander, Dr. et al
Patentanwälte v. Füner, Ebbinghaus, Finck
Mariahilfplatz 2 & 3
D-8000 München 90(DE)

(54) Process for determination of a complete or a partial contents of very short sequences in the samples of nucleic acids connected to the discrete particles of microscopic size by hybridization with oligonucleotide probes.

(57) Determination of the formula of genomic DNA, i.e. genome sequencing, by a hybridization with oligonucleotide probes (YU Patent Application 570/87) envisages the use of 100000 oligonucleotide probes and the same number of hybridizations with 6000000 of addressed sample-clones on filters in order to determine contents of oligonucleotide sequences in each clone. The process presents improvements in preparation of samples for hybridization and improvements which enable one to follow gene expression by determining partial or complete fragment sequences of genomic DNA, mRNA or cDNA. By binding fragments of genomic DNA to discrete particles (DP) of a microscopic size which are recognizable in a step of reading experimental image, the necessity for addressed samples on filters is dispensed with and this drastically reduces automatical-robotical component of the process and allows miniaturization of the entire method from a level of industrial installation to the level of laboratory instrument. Processes for binding DNA fragments to DPs recognizable in common reactions, allow elimination of cloning, i.e. DNA amplification in the host cells and, in a process of library forming, need for formation of 6000000 addressed samples in any one of the phases of a process for sequencing by hybridization.

## PROCESS FOR DETERMINATION OF A COMPLETE OR A PARTIAL CONTENTS OF VERY SHORT SEQUENCES IN THE SAMPLES OF NUCLEIC ACIDS CONNECTED TO THE DISCRETE PARTICLES OF MICROSCOPIC SIZE BY HYBRIDIZATION WITH OLIGONUCLEOTIDE PROBES

a) Field of the Invention

The present invention belongs to the field of molecular biology.

b) Technical Problem

The genome size varies from $4 \times 10^6$ nucleotides in bacteria (E. coli) to $3 \times 10^9$ nucleotides in mammals, including men. The determination of the formula, i.e. the order of nucleotides in genomic DNA, is the first-class technological challenge for the science of the end of the 20th century. It is believed that the availability of nucleotide sequence in genome would cause a qualitative rise in medicine, biotechnology and fundamental biology itself, and would beneficiently influence all fields of these sciences. As a contrast to a known method which is not effective enough for accomplishing this task, we claimed a method for sequencing by hydridization (Yugoslavian Patent Application 570/87 and Amendment No. 4521, 16.03.1988) which is adapted to a complexity of the problem. However, this method requests industrial plant and huge investments. The present solution relates to the technological improvements of a basic principle claimed which permit miniaturization, decrease of the investment costs and wider application of this method and all other applications of oligonucleotide hybridization for determination of genomic sequences.

c) State of the Art

The knowledge about parts or entire genomes on the level of primary structure as well as the possibility of following the inheritance using this information are being increasingly recognized as conditions for a more efficient and faster study of biological processes. A part of experimental investigations will be replaced by computer research on sequences obtained. It might turn out that some biological phenomena (evolutionary processes) will be accessible for study only though the analysis of genome sequences.

Two areas are recognizable in which there is an increase of organized effort to find methodological solutions which would allow the determination of primary genetical information. One is concerned with the detection of a large number of mapped polymorphic sites in genomic DNA of individual, family or population. In fact, this project means determination of a defined part of the genomic sequence which represents a specific genomic sub-entity for which we propose the name GENOGRAM. The other project deals with the determination of the entire sequence of human and other genomes. In extreme, it might mean the determination of the sequences of genomes of most species of interest and in sufficient number of individuals in each species. The first project has two basic approaches: the detection of polymor phic sequence through the specificities RE have (RFLP) or through a specificity which has hybridization with ONP. The second method has advantages in following a large number of polymorphic sites, since it does not require the determination of DNA fragment length and is easily carried out on an amplified target (PCR) or amplified ligation-hybridization reaction. In order to obtain individual genetic maps with a 1 cm resolution it is necessary to follow 5,000 to 10,000 polymorphic sites.

The second project is envisioned as a multiphase physical mapping with a final goal of determination of nucleotide sequence. Physical mapping also makes use of sequence recognition by RE and measurement of DNA fragments lengths or by the determination of contents of ONS by hybridization with ONP, and sequencing itself, according to existing methods, makes use of measurement of lengths of fragments and thus indirectly, but in the experiment, determines sequence. Two further approaches for determining experimentally the order of nucleotides are being considered as well. One is indirect and is based on the sequential removal of single nucleotides from one end of DNA fragment. The second one supposes direct experimental reading of the sequence by means of specific electron microscope. Finally, a theory of an approach has been developed in which the sequence is not arrived at directly by the experimental determination of the order of nucleotides, but the contents of ONS is found instead and then these data are transformed into sequence information by computaion work (SBH). Presently, the only realistic way for determining contents of ONS is hybridization by ONP of the same kind used in the above described methods.

In both projects and in all methods (except eventually in microscopy), one has to operate with a huge number of samples which, depending on the size and number of genomes, are to be processed amounts to between $10^6$ and $10^7$. Since each sample is subjected to one or more identical or similar reactions, there is a problem of ways of, and speed of performing of the great number of repetitive operations as well as a problem of ways and speed of gathering experimental information and storing it in computer memory. Obviously the solution for the first problem is a robotized process, and the most efficient way for gathering data is image analysis of experimental image. Speed of image analysis amounts already to one million to 10 millions pixels per second and this allows differentiating of a 10 times smaller number of point shaped objects.

Here we define "Informational Approach" for study of genome primary structure, analyze its characteristics and informational-technical requirements and give conceptual solutions for its major technological components which can substitute a miniaturized process for a massive robotized process using addressed samples. Basically, the idea is to use the mixture of samples in a form of discrete recognizable particles.

THEORETICAL ANALYSIS

Informational Approach on its Characteristics

A common characteristic of three methods which, for achieving different goals, make use of "mismatch free" hybridization by ONP is the experimental determination of the contents of a single, some or almost all ONS in specific fragments of genomic DNA. These are the method of detection of polymorphic sites, method of forming organized genome library (link-up) and SBH method. For the same targets there are methods which are, in an informational sense, based on the experimental determination of the position, i.e. the sequence of individual nucleotides or definite ONP. These methods are RFLP analysis, restrictional mapping and forming of organized libraries on the basis of restrictional pattern and sequencing by means of acrylamide gel. We define methods which make use of determination of the contents of ONS as "Informational Approach". In this approach the same principle is used for both the determination of a total sequence and of selected parts of genome (GENOGRAM) and so, in general, this approach can be defined as "Informational approach for a study and following of primary structure of genome". It has several essential characteristics.

The most essential feature of this approach is the possibility of acquiring experimental data (contents of ONS) in unpositioned samples. Since the sequence of elements is not the object of the measurements, nor is the physical distance of elements, there is no requirement for ordered spatial disposition of samples, nor should they have a defined starting position, etc.

The second characteristic is that contents of ONS can in principle be determined in a sample which in final experimental treatment occupy a micro volume or an area of optimal size. Since no physical separation is requested, such transport effects as diffusion, etc. are avoided. These effects usually impose the necessity for macro volume.

The third characteristic (which is a consequence of the preceeding ones) is a considerable density of data bits per unit of experimental volume or area. It can be defined as the possibility of achieving high degree of parallel acquisition of data.

The forth positive characteristic in this approach is the fact that data fraction, such as sample position, order of elements in the sequence, starting and ending DNA fragments or order operations, does not have to be determined experimentally, but instead, it can be replaced with informational computer processing of data which can be gained experimentally in a simpler and faster way.

The fifth feature is the increase in the number of experimental data bits (size of the matrix) at the expense of reducing amount of work necessary for the acquisition of more sophisticated and smaller matrix. This means that the burden of the process is placed on the data reading step or, in other words, on the input of more data bits in computer memory.

These characteristics allow the formulation of a concept of a miniaturized, fast and frugal process for generation of necessary experimental data. Scientific and practical needs for determination of GENOGRAM and entire genomic sequences have the same informational-technological requirements

We shall define data BITS as a discrete experimentally gained meaningful datum. Thus one data bit is a fact that the millionth nucleotide is the 5th human chromosome A. Or that X fragment of human genomic DNA contains Y ONS. A minimal number of data bits for determination of entire sequence of one mammalian genome is 3 billions or it is equal with the number of bp. Since the ultimate goal is to have all data bits stored in computer memory, for determination of a process speed it is important to know part (%) of the necessary number of data bits which can be experimentally collected and stored in a memory per unit of time.

One can assume that in future, the time for determining the sequence of a complex genome preferably cannot be longer than a year. If a second is taken as a unit of time (one year has $3.1536 \times 10^7$ seconds), then we may consider as acceptable to acquire all data bits in $10^6$ to $10^7$ seconds.

If one chooses $10^6$ seconds (12 days without interruption) as a very favourable time span, then one should obtain one part in a million of necessary data every second. It should be taken into consideration that 12 days defined in previous analysis, represent in realitiy at least a 5 to 10 times longer period because considerable part of this time is not used for effecting sequencing operations but is used instead for sample preparation, controls, etc.

How many data bits are necessary for the determination of either GENOGRAM or a sequence of one mammalian genome by determination of the contents of ONS? For sequence determination about $10^7$ separate genomic fragments (samples) and about $10^5$ ONP are needed and these figures request $10^{12}$ data bits. For GENOGRAM at least $10^4$ genomic sites or fragments are necessary for 1 cm map. It can be argued that $10^5$ dots in a genome have to be investigated because this figure is an approximate number of genes. In that case GENOGRAM would need a comparatively great number of fragments in which the contents of one or of most of ONS would have to be determined. In a such extensive GENOGRAM, in spite of a multiple use of the same ONS, the number of necessary ONS reaches the order of several tens of thousands. In practice, it is more suitable to have each ONP with a length of 7 or 8 bases and thus, with 16,000 or 65,000 ONP, to possess a system for detection of the change in any sequence. It is considered that the most efficient way is not to determine specific pairs of probes and targets but instead, to try to get more information by a simple combination of each probe with each target. Based on the analysis of this sort, it can be said that for GENOGRAM about $10^8$ data bits are necessary. We can consider as reasonable to collect on $10^5$ seconds (12 days) information for 100 to 1,000 GENOGRAMS. Roughly, it is the analysis of several patients per day or testing of a sample of 1,000 individuals of certain population within a period of two months. With such an approach as regards the requirements of molecular genetics or the future, we reach again the same number of data bits as in sequencing of a single genome (about $10^{11}$ to $10^{12}$). Therefore, extensive population and diagnostic investigations reach the amount of work necessary for sequencing of the entire genomes.

Since we established the fact that a millionth part of all data bits has to enter memory in one second, we arrive at a parameter of the crucial importance for this approach - how to produce and store a million data bits per second.

These huge technological requirements for picking up a knowledge of GENOME structure can be accomplished by developing a system for quick gaining of necessary data bits. In the next chapters one such system which makes use of the characteristics of informational approach is described.

d)Description of the Solution

Phases of informational approach

One can define four phases in experimental part of this approach, when the content of ONS is determined by ONP hybridization. After storing information related to the contents of ONS in specific fragments of genomic DNA, informational phase takes place in which the entire sequence is generated or parts thereof. These phases are:

1. Sample preparation (isolating, marking, preparation for 3.)

2. Probe preparation (synthesis, ONP bank, probe labeling)

3. Hybridization

4. Reading and storing data bits.

Three goals are defined which should be achieved either in each phase separately or in the process as a whole; samples and probes should be to the least possible extent recognizable by position (coordinates, addresses) and to the maximum extent by their innate informational characteristics; there should be the least possible number of independent hybridization reactions and ability for "reading" one million +/- ONPxTARGET data bits per second.

Samples as a system of discrete and recognizable particles

The samples can be considered in two phases: sample preparation and hybridization. Obvious solutions are preparation of addressed samples in microtiter plates and addressed hybridization dots in ordered dot-blots. One can pose the question can the use of addressed samples be avoided. This is especially important in case when the samples need not to be kept for permanent storage, or can easily be be obtained when needed, so it is easier not to keep them. The task is particularly difficult when the detection system requires more than one target molecule, i.e. amplification of genomic fragments. On the other side, one can not determine (or is convenient not to determine in order to retain parallelism) the complete contents of ONS (i.e. to hybridize all ONP) on a sample present only once in the hybridization area. Hence, it is useful to have a given genomic fragment in the large number of copies in one hybridization spot and many such hybridization spots in separate hybridi zation areas. One would like to recognize such hybridization spots, even if they are not separated as amplified genomic fragments in tubes with known marks and ordered hybridization dots with known coordinates.

As in many other methods and techniques for chemical synthesis of DNA, the answer is the use of solid support as the substitute for reaction tubes to keep the liquid samples apart. A drop of water containing the necessary number of copies of DNA fragment given is replaced by the solid particle (furtheron in this text discrete particle) which is carrying the required number of copies of the same DNA fragment attached to its surface. These particles can be looked upon as small beads of definite size and shape, similar to the ones already in use in different applications. In order to simplify description, the use of discrete particles in hybridization reaction will be presented first starting from physically separated and amplified samples obtained in a preparatory phase.

So, there is a library of genomic clones placed on microtiter plates. One adds discrete particles to each well and binding reaction takes place resulting in DNA being attached to them. Thus each liquid sample is divided into a certain number of DPs. Aliquotes of DPs from each well are mixed together and spread in the monolayer of required density. This is followed by the necessary number of fixations. In this way one can obtain hybridization areas (HA) similar to filters in dot blot procedure. Every DP represents one dot, and solid support a certain area of the filter. One can imagine a simple case in which each HA contains enough randomly displayed DPs that each clone from the library is represented at least once. The other HAs are replicas but DPs are present in different places. Every HA can in principle be hybridized and reused as classic fil ters. The main problem is to recognize DP with the same clone in different HA. The latter is probably very difficult to achieve with $10^5$ ONP required for SBH. Also, the possibility of performing many hybridization reactions in parallel is lost, if only one HA is used.

We see three principal ways of recognizing DPs which can be combined.

1) Labeling with physical attributes of DP like size, shape and color which can be differentiated in a phase of reading, for instance, during image analysis.

2) Labeling with different combinations of ONs which can be recognized as such by hybridization with appropriate ONPs. Thus, out of 20 different ONs $3 \times 10^5$ different combinations can be formed with 10 ONs each. By attaching each combination of ON to DP $3 \times 10^5$ differently labeled DPs are obtained. They can be recognized by detection of the combination given in hybridization with 20 ONPs which are complementary to ON in question.

3) Labeling (better recognition) by the use of certain fraction of ONPs on all HA. This principle is the one used in a link-up method. The requirement is that HAs can stand a testing with great number of ONPs in such a way that one part can be used for recognition. Lehrach found that 100 ONPs having the same density as 8- or 9-mer probes for SBH are suitable for recognizing overlapped cosmids in entire genomic library. In this case recognition of identical clones, not the overlapping ones, is required in a mixture of defined number of different clones. If the mixture is simplier, all clones need not be mixed at once, but separate mixes with smaller number of clones can be prepared and used to obtain subdivided HAs thus reducing the number of probes required.

With the combination of all three principles and the use of subdivided HAs one can recognize $10^7$ separate samples required by GENOGRAM and SBH. It is obvious that the use of HA divided into 10 parts and 100 "labels" per each principle allows the discrimination of this number of separate samples. In this combination scheme, one has to prepare 10000 samples with differently labeled DPs using the principles 1. and 2. However, it is obvious that maximal use of the third principle decreases the need to prepare differently marked DPs, but increases the number of required hybdridizations.

Oligonucleotide probes bound to DP : Inverse DP-SBH

Two ways of sequence determination by the hybridization have been proposed in which instead of clones, ONPs are bound to a support in form of dots. A similar system for following "expression patterns" is being developed by Southern (private communication). The problem of these methods is that for ONPs of suggested informational length of 8 bases in two cases, and 4 cases in the third one, either only a very specific information of the complex sample (8-mer located by poly A, Southern) can be determined or the sample used for hybridization must be a very short DNA, shorter than 70 bp in the one case and shorter than 200 bp in the other case. These approaches are impractical for sequence determination of the complex genomes because more than 10 millions of hybridizations are needed. On the other hand, a separate synthesis of longer probes and their deposition onto a place in HA with predetermined coordinates is probably limited to 8-10 mer lengths. However, by making use of recognizable DPs to which oligo probes are bound, it is possible to overcome these problems and such an inverse procedure (ISBH) has the potential allowing the application to genome sequencing. The base of ISBH form DPs carrying specific combination of oligonucleotide targets for recognition according to principle 2, and specific functional oligonucleotides of a length selected as in a case of generating clones by a selection (see part Direct preparation of the fragments of genomic DNA on DP). By a combined synthesis (see part on Oligo bank) all different ONPs of the particular length can be synthesized in a comparatively small number of reactions in such a manner that each ONP is on DP with a specific known combination of oligo targets. DPs prepared in this way are used for forming monolayer HA (OHA oligohybridization area) as it would be the case if DNA fragments of the certain clone were linked to them. In order that most of ONPs in OHA is represented with at least one DP, a certain amount of redundancy is necessary (ca. 10 times). By the hybridization of OHAs with the ONPs which are complementary to oligotargets from which combiantions for marking DPs are prepared (and for the most complex OHAs less than 50 probes and hybridizations are necessary, because 50 oligo targets are sufficient for preparing more $10^{12}$ combinations of 25 targets in a small number of reactions), exact position of each ONP in each OHA is established. OHAs prepared in this way with information on the position of each ONP is a "product" which can be used for very fast and simple sequence determination of the very long DNA fragments. A maximum length (DNA complexity) (L) depends on a length of ONPs (N) which have been synthetized on DP in a given HA. In order to obtain average sequence of the fragment given in a form of 10 cosmids (subfragments, SF in nomenclature of SBH) L is not to be considerably greater than $(4^N)/100$. For cosmid sequencing 11-mers are necessary, for YAC interest sequencing 13-mers. In a former case 4 million differently marked DPs are needed, in a later one 65 million. This is within a number of clones necessary for sequencing mammalian genomes by a direct SBH with 8-mer probes. If YAC clones were used in ASBH, then for mammalian genome about 1-5 thousand groups with 10 differently marked clones in each and the same number of hybridizations would be needed, while in the usual SBH 10000 hybridizations with groups consisting of 10 differently marked 8-mers. The number of syntheses and operations is in this case the same for both methods.

A process for sequence determination using OHA would comprise several simple steps:

1) random fragmentation of the sufficien mass of a given fragment of genome DNA to the lengths slightly bigger than ONPs linked to DP;

2) marking of the fragments generated; one possibility is the use of terminal transferase and one fluorescently marked nucleotide triphosphate;

3) discriminative hybridization of the marked genome fragments and OHA;

4) "image analysis" of microscopic OHA image. In these four steps sequence of ONPs in the given long DNA fragment would have been determined. By the computer processing of data according to algorithm and programmes for generating SF, either a continuous sequence of the fragment given or the sequence in a form of the limited number of SFs would have been regenerated. SBH can be successfully applied for determination of one very important part of genome information, and these are the sites with polymorphic sequence. Everything that one needs is a sufficient number of functional nucleotides which in most cases have only one target with complementary sequence in the given sample. For mammalian genome, for this purpose the most suitable are 17-mers. On the average, each tenth 17-mer should have a complementary sequence in given mammalian genome. With OHA containing $10^8$ 17-mers (less than 1/100 of all 17-mers) about $10^7$ 17-mers would be detected as positive. Since with 17-mers 17 bp can be read, OHA of this sort would allow "reading" of at least $10^8$ bp. Since it is believed that in each group of 1000 bp exists one polymorphic bp, such a OHA would allow following about $10^5$ of polymorphic sites. By analysing individuals in several generations from several families a very dense genetic map (0,1 cM) could be determined which would be useful, in a much simpler way than RFLP markers, for following in a great number of individuals for various investigations.

IBSH has several significant characteristics:

1) With a possibility of a great number of rehybridizations OHA accepts the properties of measuring instrument, or stated in informational jargon of CHIP (alternatively: sequencing card) which permits minimal sample processing.

2) A possibility for preparation of OHA of different complexity for sequencing fragments of different lengths. One can imagine OHA with 200000 9.mers for sequencing 1-2 Kb fragments, OHA with 4 million 11-mers for sequencing cosmids inserts of 50 kb, OHA with 65 million 13-mers for sequencing YAC inserts and, what is certainly most attractive, OHA with from 1 billion of 15-mers to 1000 billion of 20-mers for sequencing complete chromosomes, or genomes, or entire mRNA (cDNA) of specific tissue in only one hybridization reaction. It should be mentioned that no additional difficulty is imposed by the samples consisting of several shorter fragments (mRNA of certain tissue).

However, in case of total mRNA (cDNA) of the specific tissue, problem can arise from the different quantity of each mRNA. One possible solution for this problem is to use sufficient mass of the sample (PCR application) in order to bring the least represented mRNAs exists which has nothing to be linked to.

3) There are no reqirements for 10-100 different markers which are almost unavoidable in usual SBH in order to decrease the number of separate hybridizations. If they do exist, different markers can be sequenced by a simultaneous hybridization with sequencing card.

4) A possibility for highly specific labeling (incorporation of 100 marked nucleotides by means of terminal transferase) by means of which both the requirement for a number of ONP molecule per DP and the mass of DNA fragments being sequenced are decreased. For $10^5$ ONP molecules per one DP, in case of 15-mers, for 100 OHAs with redundancy of 10 times, it is necessary to perform 3000 synthesis, each one on the present usual scale for the synthesis of oligonucleotide in an amount of 1 mg. If 1000 molecules of ONP per DP are sufficient, then with 400 syntheses on 10 mg scale 100 OHAs with all 20-mers can be prepared.

5) A possibility for achieving great accuracy in hybridization. In order to avoid forming a great number of SFs, it is necessary to have such a ratio between L and N that, on the average, only each tenth ONP possesses complementary sequence in the given fragment of genomic DNA. On the other hand that means that chances for a larger number of sites with one non-paired nucleotide are small which represents the most difficult case for discrimination. When $L/4^N = 1/1000$ then oligonucleotide probes are approaching discriminativity possessed by unique genome probes.

The main uncertainty of ISBH is hybridization with every complex probe, especially in case of using ONPs longer than 13 bases and genome fragments larger than a million bp. The basic problem is simultaneous hybridization with ONP having two extreme GC contents. Some solutions of this problem have been already given, for instance, washing in tetramethyl ammonium chloride. Another problem is of a technical nature and has been already mentioned. It is the combination of oligonucleotide synthesis and linking of oligonucleotides already synthetized to the same DPs. Since these two reactions do not necessarily take place at the same time, solution of this difficulty does not represent huge, non-solvable practical problem. On the other hand, highly homologous and simultaneously highly repetitive sequences represent significant obstacles for this approach. In direct SBH with clones this problem has been solved by using libraries with clones of different size. Because of these sequences (LINE, SINE) a much larger number of subfragments (SF) will be formed in comparison with a case of genome with random sequence. Solution of this problem is using as big $4^N/L$ ratio as possible and/or using the existing and new information from clone systems and otehr methods for comparing generated SFs.

## Direct preparation of the fragments of genomic DNA on DP

Depending on the fact whether the detection of ONS by hybridization can be done on one or a large number of molecules of the fragment given and on the mode of the fragment amplification, one can define three possible ways of marking samples as direct mixes of DPs. Such an approach eliminates the need for preparing and maintaining of macro-separate and addressed samples.

1) Detection on an individual molecule by using DPs labeled according to principles 1 and 2 combination with recognition according to principle 3. The labeled DPs serve for discrimination of the parts of genome such as chromosomes, YACs, etc., or individuals in parallel preparation of a number of GENOGRAMS. The fragments of defined part of genomic DNA will be attached as unit molecules to specifically marked DPs in separate reactions. DPs are mixed afterwards and used to form one HA. DPs carrying the same, or more often maximally overlapped fragments will be recognized using principle 3 from the previous section. In contrast to using cloned fragments, the fragments are here rarely identical, so that groups of densely overlapped fragment are recognized. In this situation the complete contents are obtained only for a part of sequence shared by the group of fragments given. To use random groups of fragments obtained by ligation (ordering library in SBH) one needs to PCR or clone them without separation of clones. The "separation" of necessary fragments required for GENOGRAM from the rest of genomic DNA is best accomplished by a PCR reaction.

2) Amplifying by PCR. PCR can be used for preparation of genomic library of fragments with a continual length determined by a success of amplification. The length of 5 kb has been demonstrated. The procedure would require ligation of single to ends of genomic fragment mixture, the dilution of ligation products to single molecules per volume, and then their use in separate PCR reactions, for example, in microtiter wells. In this way clones of starting fragments could be obtained in vitro.

It is possible to see the implementation of PCR without the separation of individual fragments in addressed liquid samples. The requirement is that micro droplets of an amplification mixture each containing either a single fragment or none are enclosed into small spheres (pearls) formed of appropriate membranes (perhaps the semipermeable ones) together with DP conglomerates. DP conglomerates are composed of DPs of similar characteristics and should be easily separable into individual DP components under mild conditions. The use of conglomerates provides the way to prepare more DPs with the same DNA fragments which are required for multiple HA "replicas". Microsphere formation should be considered as a process for formation of fat droplets, as a statistical process with a certain degree of success, rather than a highly robotized process with high fidelity.

Every microsphere represents separate amplification reaction similar to a microtiter well. After the amplification, the reaction of binding of amplified fragments to spheres is performed in which suitable reagent for which membrane is permeable is used. The disruption of membranes and conglomerates results in a mix of DPs in which each DNA fragment is represented in a sufficient number of copies on an adequate number of DPs.

3) The separation of groups of densely overlapping fragments on DPs capable of selecting, instead of amplification of a single fragment. One can imagine separation by the selection on the basis of hybridization. One should have 10 millions of DPs each carrying specific ON. ONs will have the lengths which ensure their occurence mostly once in a genomic sequence. The ways of obtaining this number of DPs will be explained later. Random fragments (longer than finally required) obtained from a large mass of genomic DNA are subjected to action of $5'$ or $3'$ exonucleases. These fragments are subsequently randomly cut and again size selected. After selective hybridization cloning and covalence linking by ligation is performed. In this way, each DP will have bound to itself those ONs which are internally displaced for the lentgh of single-stranded end containing ON given. The recognition of DPs with the "same" fragments can be done by labeling DPs by any one or a combination of principles according to 1 and/or 2, and/or by using recognition without labeling DPs according to principle 3. This selective procedure is even more applicable to GENOGRAM where the number of samples per individual genome is 100-1000 times smaller. DPs would carry ONs of selected sequence complementary to the sequence of fragments that ought to be examined.

The procedure 1. is the most simple one in a technological sense, but the detection of hybridization on a single molecule is a difficult, still unresolved problem. The other two procedures presume many technically untested operations. On the other hand, several different, theoretically possible solution allow conclusion that preparation of defined fragments of genomic DNA, as the separate samples, can be achieved in a DP mix.

## ON bank

### Preparation of ONs (ONPs) in a mixture

The synthesis of large number of separate ONs is a considerable task if standard "gene machines" are used. However, the synthesis of ONs can be appreciably speeded up by using combination principles. This approach ensures a more rational and cheaper synthesis of smaller quantities of individual ONs. An even higher degree of rationalization can be achieved by synthesis of sufficient quantities of large number of different ONs having multiple applications and which can be used by different laboratories. This principle has been already used in the synthesis of linkers, adapters and primers. In this way ON bank would be obtained (an initiative by Crkvenjakov, Drmanac, Beattie). One can ask the question which bank would be the most useful. The answer lies in the recognition of ON characteristics that are the most suitable for major areas of their application and these are: detection of sequence by hybridization, change of existing DNA sequence and synthesis of DNA fragments (amplified fragments, subclones, clones suitable for SBH) can be performed even with very short ONPs, 8, 7 or even 6 nucleotides in length. ONPs about 20 bases long are suitable for hybridization with total genomic DNA. Primers for site specific mutagenesis and PCR are usually 15-20 mers. Even 8-mers are active primers in PCR. The procedure for DNA synthesis based on sequential joining of short blocks is being developed. We consider the bank containing all possible 3-mers to 8-mers very useful for the following reasons: (i) mentioned areas of application, (ii) technologically acceptable number of samples for bank to contain, (iii) the possibility of generating longer of ONs from shorter ones (ligation, the use of dideoxynucleotides and terminal transferase). Their total number is about 90,000. According to Beattie's calculation a bank of 8-mers (65.536 ONs) could be synthetized in less than 6 months with total investment of 3 million dollars. The cost of materials and labor for such a bank having a stock of 1-2 mg of each ON is 2 million dollars. The cost for all ONs in the stock of $10\mu g$ each would amount totally about 10-20 thousand dollars, and that is some 1000 times less than present commercial price.

The possibility of the usefulness of making ON bank on a solid support (perhaps even DP) which could be subsequently processed by machines or manually, affording specifically modified or longer ONs, has been considered too (Beattie). The use of the mixture of differently marked ONPs either in SBH or in other methods, secures the possibility of ONP synthesis as a mix, instead of forming it by mixing. For instance, 64 3-mers are synthesized each being placed on a large amount of DP and being marked differently, for example, by characteristics (fluorescence) of the molecule mediating attachment of nucleotide to DP. The mixtures of equimolar amounts of all 64 3-mers are prepared and distributed in 1024 samples. Then, synthesis of one 5-mer is continued in each part. In this way, with 1088 synthetic reactions all 8-mers can be synthetized in the mixture of 64 each.

Similar principle could be applied for the synthesis of $10^7$ DPs each carrying a different longer ON (for examples, 16-mer). These are necessary for preparation of samples in a mix, according to a principle of selective hybridization (see above under 3). For instance, 3 groups with 100 DPs in each are used (if possible 100 DPs have different physical characteristics) and in each group 100 different ONs are linked to DP. For 16-mers 2 groups are 5-mers, 1 6-mers. The same 3 groups of ONs exist as free, non-linked to DP. In 6 separate reactions involving successive permuted coupling of DP groups and groups of non-attached ONs, 6 millions DPs (a definite number of OP) with different ONs each having 16 bases would have been obtained. In this way, with 300 different starting ONs and with several reactions of permutaions and linking, a necessary number of various ONs in a mixture (in this case DP mixture) can be prepared.

Similar combinative synthesis can be applied for obtaining the bank of DPs recognizable on the base of oligotarget combinations, without or with functional oligonucleotide which can play a role for selecting fragment with a complementary sequence exists in the given sample of nucleic acids. Marking with this combination will be explained with the example of using a group of 36 different oligotargets and preparation of the combination with 18 different targets. A maximum number of these combinations if they were formed in separate reactions would be 9 billion. However, with a comparatively small number of separate reaction through a successive linking of the combinations with a smaller number of different oligotargets, it is possible to obtain essential part of all combinations of 18. If 36 oligotargets are divided into three groups of 12 in each and each group will contain 924 combinations. After linking the first 924 combinations in the same number of separate reactions, it is necessary to effect equimolar mixing of all DPs and separate into 924 tubes with combinations from another group of 12 oligotargets. By repeating the cycle once more in 2722 reactions, a mixture with 750 millions of DPs with different combinations of 18 oligotargets is obtained. Of course, DP with a specified combination of oligotargets means that each oligotarget is present in a certain number ($10^5$-$10^6$) on a given DP, DP having the same combination in a certain number in a final mixture, which depends on the fact with which mass of non-marked DPs in the process was started. Thus, when less than 10% of combination is used, a sufficient number is obtained for generating, i.e. labeling the necessary number of clones for SBH of mammalian genomes. For discrimination of the same clones, i.e DPs with the same combination, it is necessary to carry out 36 hybridizations with oligotargets complementary to probes.

For generating clones through a selection it is ultimate that all DPs in a mixture, carrying the same combination of oligotargets, possess in a certain number of copies the same oligonucleotide of the functional length selected. For 10 million clones, taking into consideration that process efficiency will be 10%, it is necessary to have 100 millions of DPs with different combination and different functional oligonucleotides. For ISBH that number can be within a range of $10^6$-$10^{12}$ depending on the lengths of DNA which are to be sequenced in one reaction. Besides, with ISBH one has to know which oligonucleotide is bound to which combi nation on the same DP, which is not the case with selective forming of clones. Also, for ISBH one has to use a large amount of oligosequences of the specific length.

The principle of preparation of these DPs will be explained with the example of the synthesis of all 15-mers in three cycles. Basically it is only an extension of the procedure for marking with oligotarget combinations. In any case, one third (5-mer) for all 15-mers. The three groups should be formed, each containing 1024 combinations (this is a number of different 5-mers), starting from the smallest number of different oligotargets. In this case, it is a part of combinations of 6 oligotargets from the group of 13 oligotargets. A total number of oligotargets is 39, i.e. this is a number of necessary hybridizations for DP discrimination. In each first combination from the group the first 5-mer is added (for instance, AAAAA) and so on until GGGGG is reached. At this moment, to a combination from the first group non-labeled DPs are added, and then oligotargets and given 5-mer are linked to DP; DPs are mixed and equimolarily distributed into the combinations of the second group. In this step, oligotargets are linked to DPs as separate molecules, and 5-mers affording 10-mers. In each of 1024 reactions of the second group 1024 10-mers are synthesized, i.e. all 10-mers are synthesized. By repeating the same operations in the third cycle all 15-mers in 3072 reactions are obtained in such a manner that one knows exactly which 15-mer is on DP with which specific combination of oligotargets. 5-mers are not necessarily added as the finished units, but instead synthesis thereof can be executed in the given 3072 reactions. When a complete procedure is performed in 5 cycles with 64 reactions, then totally only 320 independent reactions are required. In this case, it is necessary to divide 40 oligotargets in 5 groups with 8 combinations in each group with 4 oligotargets. These examples illustrate the power of combinative synthesis in which the number of operations grows by arithmetical and number of syntheses by geometrical progression.

Taking into consideration possibilities of combined synthesis, bank forming and synthesis in a mixture, one can conclude that a number of syntheses and manipulations is not necessarily large and, consequently, ON price should be directly related to the mass, i.e. cost of the material. The use of DPs as a substitute for dot-blots offers in this sense considerable advantages because it requires a lower amount of ONP. For the same target density per area a much smaller amount of target per DP is necessary, than per dot. The total area of HA is also much smaller and this decreases the necessary amount of hybridization buffer, i.e. ONP mass. If DP diameter is $4\mu$m, then the area of its maximum section is ca. 10 $\mu$m$^2$. When dot area is 1 mm$^2$, the ratio is 1:10$^5$. Based on the calculations that in forming of random monolayer 10-fold more DPs must be used in order that each one is represented at least once in HA, and that in this case utilization of space is only 10%, ratio would be 1:1000. Speaking in absolute numbers, in case of DP area of one HA would amount to 10x10 cm, while in dots would be 1x1 m. Assuming that one HA can be used for testing 1000 ONPs (mixture of 10-100 ONPs x 100-100 washings), the total area is 1 m$^2$, vs. 1000 m$^2$. In the first case necessary amount of each ONP in SBH can be calculated in the following manner. One ONP has a target in each tenth clone and since ten times more of DP is necessary because of random sampling, the total number of DPs with which one ONP is hybridized equals the number of clones (10 millions). For a signal detection on one DP using CCD cameras, it is sufficient to perform labeling with less than 1000 fluorescent molecules (private communication). If one supposes that in hybridization only 0.1% of ONP is made use of, then one needs 10$^{13}$ ONM molecules for hybridization with all clones. Since 1 $\mu$g of 8-mer ONP contains about 3x10$^{14}$ molecules, then such a mass of individual ONP is more than sufficient. The dot system would probably request larger mass of the order of 1 mg. The dollar savings per one genome (or 100 GENOGRAMS), according to Beattee's prices for a library, would be about a million US-$ in a transition from dot to DP system.

Detection of ONs contents on a level of one DNA molecule

If one restricts himself to consideration of hybridization as a procedure for determination of ONs contents, the problem of detection of single target molecule has two components. The first is the possibility (successfulness, efficiency, probability) of occurence of the hybridization event with a single target molecule (there can be an excess of ONP) and the second is the detection of the hybrid obtained. Since no efficient or simple procedure for detection of single molecule hybridization has been developed so far, there is no knowledge of this reaction either. One can assume that the event of single molecule hybridization occurs with a certain probability (in a defined % of trials). The detection of such an event can be of two kinds. In the first, the detection of the signal is produced by the marker on hybridizing probe (e.g. fluorescent molecule, enzymatic activity), even if later amplified in various ways. In the second kind, the hybridization event is amplified itself. Its logic is the same one used in all exponential doublings in natural and in vitro amplification reactions (cell division, DNA replication, PCR, ligation-amplification reaction (LAR)). The total amount of product is k x n$^c$, where n is usually 2 and represents the amplification factor, c is the number of cycles and k the efficiency factor. For GENOGRAM determination one can use LAR, since the basic requirement of the method is obeyed, and that is the previous knowledge of sequence or the small number of its variants. LAR is more difficult to apply to SBH of unknown sequence. ON which is the reporter of hybridization event (usually carries biotin) would have to be very short in order to use all theoretically possible sequences in a mixture (probably 4-5 mer). In addition, LAR has a problem of how to localize ligation product on dots or DPs on which it is formed. If the problem of local fixation is resolved, one can avoid the requirements of the specificity of ligation reaction and reporter molecules. The scenario for simple amplification hybridization could look this way:

DPs carrying the capability of binding ONPs having a specific chemical group on one end, and a single target are prepared. Then one hybridizes with an ONP that is both complementary and carries the chemical group. After discriminative hybridization and washing, the reaction of denaturation and binding of ONP to DP is performed. Care should be taken of that this can be "possible only" with ONP which is hybridized to the target on a given DP.In this way DP having a positive hybridization would form two targets. The hybridization reaction is repeated in which both the starting and complementary ONPs are used (synthesized target). In a new cycle of denaturation and binding of hybridized ONPs, the number of targets is doubled. One repeats these cycles until a detectable number of ONPs is bound to DPs. It is interesting to note that after the first cycle which should be with short ONPs (8-mers for SBH), one can switch to hybridization with longer probes and targets quite independent from the primary target. The discrimination is thus easily achieved in a high number of cycles. This is accomplished by the use of synthesized targets which are longer than primary ONP and by the use of one additional ONP complementary to a synthesized target.

This scheme is just a theoretical possibility in detection of single molecule hybridization and does not presume experimental feasibility. In any case, the detection of hybridization (or in general) on the level of a single molecule being a process of small probability of positive outcome can be treated statistically as a result of trials on a large number of DPs with the same target or a larger number of trials with same ONP on the same DP. This larger number of trials on the one DP is integrated in many cycles of hybridization. In this case the positive hybridization is recognized within a wide span of signal intensities above a certain threshold. This is similar to the situation when in dots or DPs is a large difference in the molarity of amplified targets.

Image analysis

The possibilty of different labeling of ONPs for use as a group

It has been established that in the informational approach of desirable efficiency for determination of a genomic sequence, one needs to store in a computer memory $10^5$ to $10^6$ bits of binary information per second. Since the matrix of informational genomic approach consists of $10^7$ targets x $10^5$ ONPs, this speed can be theoretically attained at the two extremes: by reading submatrix of 1(10) targets x all ONPs per second, or $10^5$-$10^6$ targets x 1 ONP. Of course, all other more or less square submatrices are possible as well. From a practical standpoint it is simpler not to prepare those matrices which are based on the extremes of the one or other component. For instance, if only one matrix with a single ONP is used, one would have to perform 100000 separate hybridizations. On the other hand, it is difficult to perform simultaneous hybridization followed by recognition of 100000 ONPs. Therefore, parallel formation of many submatrices of the type $10^4$ to $10^5$ targets x 10-100 ONPs seems to us as most rational way to proceed. The parallelism can be of the two kinds: a formation of all 100-1000 submatrices with the same group of ONPs on a single HA, and simultaneous hybridization in separate vessels on HA "replicas" with many different ONP groups. From a standpoint of the number of separate hybridizations, it would be more favourable to use groups of 100 ONPs which would require only 1000 separate hybridization reactions and probably as many separate ONP syntheses (see section on ONP synthesis in a mix). On the other hand, $10^4$-$10^5$ targets require about million pixels in electronic cameras by means of which efficient image analysis is achieved. CCD cameras can have from 650000 to 1.3 million pixels of about 10 x 10 $\mu$m and, therefore, matrix suggested can be looked upon as a single picture. The size of pixels and of DPs are not directly dependent on each other because of the possibility of using optical microscope. For image analysis one can use DPs of 0.1 $\mu$m size.

The speed of image analysis possessed by present CCD cameras is 50000 pixels in a second and this is about 20 times slower than the requirement of 1 million pixels per second. The speed indicated includes "photographic recording", digitalization and storing in a computer memory. Probably technical characteristics are limiting factor rather than theoretical limitations. With a more powerful device fast digitalization can be attained, especially when no large digital resolution is needed. Kodak announced a camera which reads 1 million pixels with 8 bits (discrimination of 256 levels of signal intensities) in 10 shifts (10 different images) per second. On the other hand, it should be pointed out that hybridization image does not have to be seen or reconstructed on a display, and this fact means a time saving.

In the most suitable submatrix there is a formidable problem. It is a simultaneous hybridization with 10-100 ONPs, and to achieve this one needs that many labels recognizable in a mixture. One should also keep in mind that in SBH mathematical expectation is that each probe will hybridize with 10% of clones (or even less in GENOGRAM). This means that use of the mixture of 100 ONPs would hybridize approximately 10 ONPs per each dot or DP. There are two experimentally confirmed approaches which can influence solving this problem. One is the use of different fluorescent molecules, and the other is gas chromatography coupled with mass spectroscopy. In the former it is difficult to imagine the use of more than 10 different fluorescent molecules and also for their detection (on a single DP) one needs the exciting light of different wavelengths or filters. Every change of wavelength or filters is an additional physical operation, making optimization possible only in hybridization and not in image analysis. However, extreme precision and sensitivity of CCD cameras (down to two photons per second) lend to this approach great possibilities. The second approach can potentially discriminate even 1000 labels, but is suitable (or possible) only on single, or a small number of samples. For this methodology to work one would need to develop technology of parallel acquisition of total data from 100-1000 samples per second. This is difficult to blend with the use of unordered microsamples in form of DP.

The most simple for image analysis is to discriminate between a great number of objects on the basis of their physical characteristics such as size, shape and color. These characteristics are reduced to different photon patterns in contrast to defined photons emitted by fluorescent molecules. This permits the recognition of an "unlimited" number of non-overlapping objects. Therefore, the recognition of hundreds of physically different DPs in image analysis must be simple. One can ask the question whether this principle of target labeling can be used for ONP recognition (labeling) as well. Two principles can be applied:

1) ONP carries a physical entity recognizable by optical microscope and thus usable in image analysis, and

2) ONP carries a chemical entity which can be used after hybridization as an initiator for localized formation of a specific physical entity.

The most simple application of the first principle is to double DP system in which ONPs are bound to DP which can be mutually discriminated according to physical characteristics. Positive hybridization would lead to rosette formation. The target DP would be surrounded with DPs carrying ONPs whose complementary sequences are present in a given target. This principle of the visualization of bimolecular recognition reaction is employed in immunology where positive antibody-antigen reaction is recognized by the rosettes formed by the appropriate cells. The basic difficulty is whether ONP hybridization can lead to the formation of a sufficient number of chemical bonds the energies of which are strong enough to hold the two DPs together. One should not forget that the applied system must allow simultaneous discriminative hybridization on the level of one base pair mismatch.

The second approach does not require linking of DPs by chemcial bonds formed in hybridization. Its problem is the way how an initiating event on ONP can be transformed into a locally recognizable physical character. One should probably take advantage of a local concentration of a certain reagent (for instance, a certain metal ion). One can rephrase the question in the following way: how can one transform in one or several reactions 10 different ions, distributed locally, into the localized, physically different entities. One can speculate on the ionic initiation of chemical interactions on the surface of target DPs with specific DPs added in the system. The other possibility is the initiation of local forming of specific, recognizable microcrystals.

Advantages of the solution described

These solutions represent an attempt to define a more rational approach to the development of the methods necessary in a resolution of central problems of molecular biology such as determination of genome primary structure, determination of regulation and self-regulation of biological systems, treatment of cancer and others. Theoretical treatment of the problem and the comparison of the properties of all theoretically possible approaches has a goal to discard some methods and procedures as inefficient, or impossible, and to initiate and encourage the development of the procedures which are more efficient. Thus, one can pose the question why the PCR reaction has not been "discovered" earlier when all its material components were known. It is tempting to assume in the retrospect that a thorough theoretical analysis of the ways of the amplification of single DNA fragments or fragment libraries could have predicted PCR with its now plainly obvious advantage. Would the existence of such theoretical concept have led to earlier application of PCR? Due to its complexity and size, the human genome project inevitably needs theoretical treatment of methodological requirements and the ways they can be satisfied. Empirical discovery of more efficient approaches is not possible, because genome cannot be subjected to minor experiment.

The INFORMATIONAL PRINCIPLE defined here is based on the use of ONs words as in a case with the efficient algorithms for sequence comparisons. For the moment, for determination of ONs content there are two (with experimental confirmation) natural molecular processes: recognition on the basis of complementarity of NA and specific recognition used for some proteins. The first process is more general because any sequence can be recognized and probably is easier due to NA stability. In light of this, we believe that ONP hybridization will have a central role in compilation of genomic sequences. The basic informational characteristic of ONP hybridization is a determination of ONP contents and this is probably the best way for recognition of ONs.

The fundamental principle allow the technological advantages of unbroken parallelism (the samples travel together from genomic DNA to IMAGE ANALYSIS) and amplification cascades (100 HA x 10 genomic parts x $10^{5-6}$ DP) x 10 washes x 100 ONPs/hybridization x 10 days = $10^{13}$ unit information data). This can be called a parallelism of parallel processes, i.e. quadratic parallelism. A small number of operations is performed in each step, but due to multiplication and not summation of gains of unit information bits per step, the total yield of information bits is enormously large. One can also make use of the technological advantage secured by maximization of the use of resources and materials prepared beforehand that are identical for different samples and the minimization of sample specific treatments. This functional cassette preparations are independent (and usable for any) of the individual object of a species given, i.e. individual genome. The consequence is preparation of ONPs containing integrated information of type - basic element x position, usable for any DNA. This also results in DP preparation integrating the addressing information, so that it needs not be determined by robotic positioning operations. The computer software package for generation of seuqence which was once made, is reusable on new data sets without the intervention of the scientist-experimentalist.

In the final analysis, the INFORMATIONAL APPROACH provides the decrease of experimental requirements at the expense of the informational computer work. Based on the several described conceptual solutions and possible, or eventually possible, practical procedures, one can estimate a potential for decreasing experimental requirements. Thus, the experimental surface area and the ONP mass are decreased by about 1000 times. The corresponding decrease can be expected and for the necessary mass of the samples which would be probably required in a classical sequencing and in a dot-blot system with the vessels for clone cultivation which are at least 100 $\mu$m each per clone. The total volume for 100 million samples would be of the order of several tons, and in invented micro-hybridiazation several liters. Even more important than space and material savings are reductions in the number of robotic operations. A robotic hand with 10000 pipetting fingers needs 1000 operations to make one filter with 10 million dots. Using the DP system, a robotic hand with several pipetting units (10-1000) can perform an analogous task in a single operation. All this can lead to a miniaturization of "genomic installations" onto a size of the bigger laboratory instruments of nowadays.

The DP system in essence represents the imitation of multitude of biochemical reactions occuring simultaneously within a single cell. Specificity and discreteness of cellular reactions are based on enzyme actions whose informational properties are imitated here by DPs. The use of DPs requires an at least 10-fold increase in the number of unit information bits, but time and labour investments (preparatory and robotic operations) for obtaining the complete data set are reduced several times. The several examples which follow are intended to show how one can transfer the center of operations to IMAGE ANALYSIS and thus, to make the most efficient step. In a robotized dot system, every DNA fragment is represented in each HA only once. In DP system this certainty is replaced with the probability that each clone is represented at least once in HA. This imposes the 10-fold increase in a total number of DPs. On the other hand, this and even bigger increase allows the tolerance of imperfect hybridization on individual DP, i.e. statistical determination of positive hybridization. This means in the last instance, the reduction of required experimental performance levels. Therefore, the DP based hybridization and signal reading procedures must tolerate the libraries consisting of a larger number of fragments which, in turn, allows the use of smaller number of shorter ONPs. This is especially evident in GENOGRAM application. Instead of specifically choosing of 10000 pairs of primers and ONPs, it is more efficient to perform hybridization of all amplified fragments with all ONPs. The advantage is further strengthened by the realization that the ensuing surplus of information means the higher accuracy in polymorphism determination as well as the possibility of detection of new mutations, both of which can be of considerable diagnostic value. By switching the emphasis to the IMAGE ANALYSIS or, in other words, by decreasing a volume of experimental work, it is possible to obtain large numbers of detailed GENOGRAMS according to the same principle as in determination of the entire genomic sequence.

The use of these characteristics of INFORMATIONAL APPROACH provides for as a final result, besides miniaturization, a greater speed in comparison with the processes requiring experimental gathering of various and more complex data bits.

It is interesting to attempt to outline in a comparative analysis the advantages and disadvantages of INFORMATIONAL APPROACH for genomic sequencing versus the three procedures which use EXPERIMENTAL APPROACH consisting of position determining methods. The standard method used up to now, which is based on the finding of the position by measuring the length of DNA fragments has two requirements which are almost certainly excluding it as a method of choice. These are the practical impossibility of miniaturization and need for use of amplified fragments of genomic DNA. The other two methods, which like SBH or other procedures using the INFORMATIONAL APPROACH have not been experimentally verified so far, do not impose these requirements. The tunneling electron microscopy, used as tools for direct reading, is an inherently miniaturized procedure which does not require amplification of a DNA fragment. On the other hand, the sequential removal of base by base from one end of DNA fragment, followed by continual separation by flow and efficient registration in passage by the detector, for practical reasons almost certainly requires the use and detection on a level of one molecule. Probably, it is very difficult, or may be even impossible, to synchronize removal of the same nucleotide on the level of moles of the fragments. It can be imagined that this approach can be miniaturized and made parallel, since instead of addressed reactions, multiple microtubes can be used ensuring discreteness. In addition, separation of removed nucleotides by a water flow does not require macro-separation, as this is the case in separation of DNA fragments, with an accuracy to the level of one base acrylamide gel. The main requirement of this approach is precision and speed of detection of single events, especially parallel detection in large numbers of microtubes. The question is, can the the use of lasers and fluorescence labeling combined with pixel based image analysis allow the acceptable data acquisition speed with non-prohibitively complex equipment.

In any case, both procedures are relying on achievements in physics, while INFORMATIONAL APPROACH is exclusively based on biochemical, molecular processes. That is so because in SBH, as indicated here, one can arrive at the experimental image and IMAGE ANALYSIS with minimal technical requirements. Since there is an indirect detection of molecular reactions, SBH does not have to have atoms and since it does not use position information, SBH does not require any physical ordering of reaction allowing the use of amplified fragments.

All methods have a common last step including image analysis of "experimental image". The question is what is the ease of arriving at this analysis. It appears to us that SBH is more adapted, more efficient for sequencing a large number of complex genomes. Due to its requirements for preparation of ONPs and DPs SBH is valuable, and perhaps more efficient for sequencing on a large scale in comparison with other methods. The reduction of individual genomes on a common denominator - ONS, allows the use of informational work after image analysis for sequence generation. The entire work in non-informational approach is of the experimental character.

## Claims

1. Process for determination of partial or entire nucleic acid sequence by the hybridization of the samples in a mixture, **characterized** in that multiplied or synthesized or separated DNA or RNA molecules in separate reactions are bound to discrete particles (DPs) of microscopic size which can be discriminated according to physical and chemical characteristics thereof, DPs are mixed, then they are hybridized with an individual or with a group of probes which are natural or multiplied or separated DNA or RNA molecules, and the result of hybridization on the individual samples is detected either by an ordered flow of DPs one by one as they are passing by a detector, or by forming a monolayer spread of DPs which permits detection by image analysis.

2. Process according to Claim 1, **characterized** in that natural or multiplied or synthesized or separated DNA or RNA molecules are linked in separate reactions to discrete particles (DPs) which can be, but not necessarily, discriminated according to physical and biochemical characteristics thereof, DPs are mixed, the mixture spread into one big or several smaller separated areas on a solid support, after which DPs are fixed to the support.

3. Process according to any of the preceding claims, **characterized** in that from the same number of preparations of different oligonucleotides of the known formula different mixtures are prepared using combinations of a certain number of starting oligonucleotides, each mixture is bound in separate reactions to DPs, and DPs carrying the same combination of oligonucleotides are recognized through a hybridization with oligo probes which are complementary to starting oligonucleotides.

4. Process according to any of the preceding claims, **characterized** in that in a small number of the reactions DP mixtures carrying specific combinations of oligonucleotide targets, which are used for recognition of DPs, are made, oligonucleotide targets are divided into a specific number of groups and combinations from each group are formed and placed in the separate tubes with a specific number of different oligonucleotide targets from the given group, then either identical DPs are added in the tubes with combinations or an equimolar ration of DPs can be discriminated in each tube according to physical characteristics thereof is added, binding of oligonucleotide targets to DP is carried out, DPs from all reactions are mixed either equimolarily or in specific cases in the specific ratio, then they are divided into the tubes with combinations of oligotargets from another group, and the given cycle of mixing and dividing DPs and binding the new combinations of oligotargets to DPs is repeated as many times as there are the number of groups of oligotargets.

5. Process according to Claims 3 and 4, **characterized** in that DP besides certain combinations of the oligonucleotide targets or some other marker contains functional oligonucleotide of defined length of the functional oligonucleotide which is common for all of the DPs in a given reaction is synthesized, before, during or after the binding of the given oligotarget combination to DP or other marker, in each cycle in a given tube with the specific oligotarget combination in that way that in a successive reaction of the binding of the oligotarget, combiantions, or other markers, continues the syntheses of a needed part of the functional oligonucleotide on the part synthesized in the previous cycle entire given part which is synthesized in the independent process binds to the part synthesized, or bound in the previous cycle, that is, binds to the DP in the first cycle.

6. Process according to Claims 1, 2 and 5, **characterized** in that the hybridization surface (area) consists of solid support with a fixed monolayer spread of DPs with characteristics made in a manner that in DPs is represented the informative part of or all possible different oligonucleotides of certain length/s, the position is determined of DPs with each and every functional nucleotide by hybridization with oligotargets which are used for forming of the combinations on the DPs, or in some other way, if the DPs are not labeled with the oligotarget combinations.

7. Process according to Claims 1 and 6, **characterized** in that a sufficient mass of the given nuclear acid sample whose total complexity is not too high for the functional oligonucleotide length in a given hybridization surface (area) is cut in a random process in very short fragments, although longer than the functional oligonucleotides, generated fragments are then labeled, discriminative hybridization with the hybridizational surface (area) with the characteristics is performed, in the process of microscopic image analysis of the given hybridizational surface (area) is determined on which DP the positive hybridization did take place, obtained information, on the basis of the information from the given surface (area) on the position of the DP with the given functional oligonucleotide, is translated into content of the oligonucleotide sequences in the given nucleic acid sample and finally by computer analysis partial or total nucleic acid sequence in the given sample is obtained.

8. Process according to Claims 6 and 7, **characterized** in that for the determination and tracking of the heredity of the large number of the genomic or gene polymorphic sequences for identification of the person, determination of the relatedness or evolutionary distance, detection of the changes on the genome and genes, prenatal and postnatal prediction of the phenotype characteristics, determination of the biological function of the individual genes or gene complexes by determination of the sequence, a certain fragment, or total human DNA of the person, or individual cell, or mRNA, or cDNA of the certaun tissue or group of the tissues, is processed and hybridized with hybridization surfaces that are containing sufficient number of different functional oligonucleotides, so that in the largest number of cases have complementary sequences on a single point in a given nucleic acids sample, and from pattern differences between samples from individuals, the poylmorphic sequences are determined, whose haplotype combinations in a new sample could be determined by applying the same procedure.

9. Process accordsing to Claim 1, **characterized** in that oligoncletide probes in a certain number of moles are bound to visually recognizable discrete particles (DP) that are different from prom to probe so that one can apply a mixture of oligonucleotide probes so that one can apply a mixture of oligonucleotide probes, and hybridization event after suitable hybridization washing is recognized as a rosette of discrete particles containing corresponding probe bound to discrete particle or a point on the solid support where a target containing complementary sequence is placed.

10. Process according to Claim 1, **characterized** in that for identification of the uncloned genes or gene families and parallel investigation of the place, time and modulation of the total gene expression by means of determination of the sequence of the mRNA or cDNA prepared from a certain tissue, a certain cell cultures, a certain tissue at a certain stage of the ontogenic development, or cell cultures or tissues after the influence of certain environmental agents, are hybridized with the sufficien number of a single or groups of the oligonucleotide probes of a length of a 4 to 12 bases and on the basis of the detected oligonucleotide sequence content, the pattern (profile, stage) of the expression or relatedness of the genes is determined.

11. Process according to Claim 11, **charcterized** in that for applications for determination of the partial or the total sequence, the genomic DNA, mRNA or cDNA library of the given biological sample (DNA, mRNA or cDNA) are bound to the DPs and hybridized with the part, or all of the oligonucleotide probes of the length of the 4 to 20 bases, and on the basis of the detected content of the oligonucleotide sequences by means of the computer processing, a partial or total sequence of the individual clones is obtained, and thereby the sequence of the given nucleic acid sample.

12. Process of forming of the library of the discrete particles (DPs) for determination of the partial or the total nucleic acid sequence by hybridization of the samples in a mixture according to Claims 10 and 11, **characterized** in that DPs are carrying a single molecule or a certain molarity of the same or mostly overlapped fragments of the genomic desoxyribonucleic acid (DNA) or ribonucleic acid (RNA), so that the library contains a certain number of discrete particles with the same nucleic acid molecules, and as the whole the sufficient number with different molecules so that the contents of the sequences in a starting biological samples are represented, by using the mixture of the DPs containing functional oligonucleotides whose sequences appear only once or are non-existent in a starting nucleic acid sample in which every sequence is represented in a large number of moles, by means of hybridization process, a sorting of the nucleic acid fragments is performed, as well as their fixation to the DPs afterwards, so that all of the DPs with the same functional oligonucleotides conatin fragments with the same, mostly overlapped or very similar sequences from libraries.

13. Process according to Claim 12, **characterized** in that to the genomic fragments of appropriate sizes are enzymatically bound on both ends the same or different short fragment of the desoxyribonucleic acid (DNA), followed by sufficient dilution of the fragments, so that the forming is permitted of the separate samples containing a single, or no molecules, and in vitro enzymatic amplification is performed by polymerase chain reaction using primers that are complementary to the ligated DNA fragments, or by using of ribonucleic acid (RNA) polymerases, if the ligated fragments are promotor sequences.

14. Process according to Claim 12, **characterized** in that the library formation of the discrete particles (DP) is performed by using amplification reactions in hich conglomerates of the DPs by random process are enclosed with the certain amount of the amplification mixture with one or none genomic desoxyribonucleic acid (DNA) fragments, or complementary DNA or ribonucleic acid (RNA) into membranes impermeable for the macromolecules, followed by an amplification, DNA is fixed to the DPs, followed by disruption of the membranes and conglomerates resulting in the individual DPs mixture in which majority of the DPs contain a large number of copies of the same fragment of the genomic DNA, complementary DNA, or RNA.

15. Discrete particles according to Claims 1 to 14, **characterized** in that they possess the same or different physical or chemical characteristics and are containing combinations of the different oligonucleotides of the known formulas that are represented either as an individual molecule of each, or the certain molarity of each, so that given oligonucleotide combination serves for discrete particles recognition by hybridization, or in any other obvious way.

16. Discrete particle (DP) conglomerates according to Claim 14, **characterized** in that DPs in conglomerate have the same or similar physical or chemical characteristics and are bound together by weak physical or chemical bonds, thus enabling easy disassembling to individual DPs, the discrete particles between different conglomerates are recognized by size, shape, colour, chemical properties, or by oligonucleotide combinations or in any other obvious way.

17. The mixture of the discrete particles (DPs) according to Claims 3 and 6, **characterized** in that every DP in the mixture contains one functional oligonucleotide as a single molecule, or in a certain molarity, and DP is represented in the mixture once or several times, DPs with the same oligonucleotide possess the same physical or chemical characteristics, and discrete particles containing a different oligonucleotide can, by physical or chemical characteristics, be identical or different by size, shape and colour, or can contain different oligonucleotide combination, or in any other obvious way.

18. Genomic DNA fragments, cDNA, cRNA molecules and their sequences, **characterized** in that they are identified, or isolated, or that their sequence is determined, by using processes according to Claims 1 to 14.